# EUROPÄISCHE PATENTSCHRIFT

(11) **EP 0 563 971 B1**
(45) Veröffentlichungstag und Bekanntmachung des Hinweises auf die Patenterteilung: **23.10.1996**
(21) Anmeldenummer: 93105448.0
(22) Anmeldetag: 01.04.1993
(51) Int. Cl.: A61F 13/15

(54) **Wegwerfwindel**
Disposable diaper
Couche à jeter

(30) Priorität: 01.04.1992 JP 80052/92
(43) Veröffentlichungstag der Anmeldung: 06.10.1993
(73) Patentinhaber: UNI-CHARM COMPANY LIMITED, Kawanoe-Shi, Ehime 799-01 (JP)
(72) Erfinder: Tanji, Hiroyuki, Ehime-ken (JP); Wada, Ichiro, Kawanoe-shi, Ehime-ken (JP); Ono, Yoshio, Kawanoe-shi, Ehime-ken (JP); Soga, Hiroyuki, Kawanoe-shi, Ehime-ken (JP)
(74) Vertreter: Sperling, Rüdiger, Dipl.-Ing.

(56) Entgegenhaltungen:
- EP-A- 0 241 925
- FR-A- 2 572 902
- FR-A- 2 668 364
- US-A- 4 662 877

## Beschreibung

Die Erfindung bezieht sich auf eine Wegwerfwindel, welche zum Absorbieren und Halten von Ausscheidungen aus dem menschlichen Körper verwendet wird.

Die japanische Gebrauchsmusteranmeldungs Nr. 1974-120439, offenbart eine Windelhülle mit einer in ihrem zentralen Bereich angeordneten oberen Schicht, die eine Öffnung hat, welche sich in Längsrichtung der oberen Schicht länger erstreckt als in ihrer Querrichtung, wobei die Öffnung entlang ihres äußeren Randes ein in Längsrichtung dehnbares elastisches Teil aufweist, um eine geschlossene ringförmige elastische Linie zu bilden. Die japanische Patentanmeldung Nr. 1986-41304 offenbart ebenfalls eine Wegwerfwindel mit einer oberen Schicht, die in ihrem zentralen Bereich eine sich in der Längsrichtung länger als in der Querrichtung der oberen Schicht erstreckende Öffnung aufweist, bei der die Öffnung entlang ihren seitlich gegenüberliegenden Seitenrändern elastische Teile aufweist.

Bei diesen die Öffnung aufweisenden Windelhüllen und Windeln fließen Ausscheidungen durch die Öffnungen in eine Tasche, die zwischen der oberen Schicht und einer weiteren unterhalb der erstgenannten oberen Schicht liegenden definiert ist, und wird dort gehalten. Hiermit können Hauterkrankungen, die häufig auftreten, wenn die Haut des Benutzers mit Ausscheidungen verschmutzt wird, welche über die oberste obere Schicht hervortreten, zuverlässig verhindert und Unbehaglichkeitsgefühle des Benutzers gelindert werden.

Vom Standpunkt der öffentlichen Hygiene ist es erwünscht, benutzte Windeln wegzuwerfen, nachdem feste, in der Windel enthaltene Ausscheidungen in Toiletten entfernt wurden, wobei diese Praxis aufgrund öffentlicher Hygiene generell befolgt wird. Bei den voranstehend erwähnten, vorbekannten Windeln, die die Öffnung aufweisen, ist es jedoch schwierig, Ausscheidungen in Toiletten zu entfernen, da die Ausscheidungen in die Tasche fließen.

Die Vorgehensweise des Entfernens fester Bestandteile kann vereinfacht werden, durch Wegziehen der obersten Schicht von der Kante der Öffnung, um ein Großteil der oberen Schicht aufzudecken, die unterhalb der obersten Schicht angeordnet ist. Jedoch ist es durch die Öffnung, deren Ränder durch endlose elastische daran als geschlossen kreisförmig befestigte Teile gemäß der Windelabdeckung, wie sie in der japanischen Gebrauchsmusteranmeldung Nr. 974-120439 veröffentlicht ist, schwierig, die oberste Schicht von dem Rand der Öffnung zu entfernen, wenn nicht ein geeignetes Werkzeug, wie beispielsweise ein Messer oder eine Schere verwendet wird. Entsprechend dieser Unbequemlichkeit ist es zu befürchten, daß Windeln mit darin zurückgelassenen oder unvollständig entfernten festen Ausscheidungen wie gewöhnlicher Müll weggeworfen werden.

Es ist eine Aufgabe der Erfindung, eine Wegwerfwindel bereitzustellen, die so verbessert ist, daß der gesamte äußere Rand mit elastischen Mitteln elastisch gemacht ist, jedoch die oberste Schicht in einfacher Weise von dem Rand der Öffnung abgezogen werden kann, um die andere obere Schicht, welche unter der obersten Schicht liegt, aufzudecken und dabei das Vorgehen des Entfernens zu vereinfachen.

### Zusammenfassung der Erfindung

Die voranstehende Aufgabe wird gemäß der Erfindung bei einer Wegwerfwindel mit einem Integral-Laminat, welches zusammengesetzt ist aus einer flüssigkeitsdurchlässigen ersten oberen Schicht, einer flüssigkeitsundurchlässigen unteren Schicht, einem flüssigkeitsabsorbierenden Kern, der zwischen der ersten oberen Schicht und der unteren Schicht eingelegt ist, und einer flüssigkeitsabweisenden, über der ersten oberen Schicht liegenden zweiten oberen Schicht, wobei die zweite obere Schicht in ihrer zentralen Zone im wesentlichen mit einer Öffnung versehen ist, welche sich in der Längsrichtung länger erstreckt als in der Querrichtung der zweiten oberen Schicht; wobei die zweite obere Schicht entlang ihres äußeren Begrenzungsrandes an die erste obere Schicht gebunden ist; und wobei in Längsrichtung elastische Teile an der zweiten oberen Schicht entlang des äußeren Randes der Öffnung angebracht sind, dadurch gelöst, daß
die elastischen Teile erste und zweite elastische Teile aufweisen, welche nicht miteinander verbunden sind, und daß im wesentlichen der gesamte Rand der Öffnung elastisch gemacht ist durch das Anbringen dieser ersten und zweiten elastischen Teile im wesentlichen entlang der jeweiligen Hälften des Randes.

Vorzugsweise sind die in Längsrichtung gegenüberliegenden Enden der ersten und zweiten elastischen Teile an den in Längsrichtung gegenüberliegenden Enden der Öffnungen überlappend, berührend oder sich annähernd angeordnet.

Vorzugsweise sind die ersten und zweiten elastischen Teile durch den äußeren Rand der Öffnung abgedeckt.

Vorzugsweise weist jedes der ersten und zweiten elastischen Teile zumindest einen Gummifaden auf.

Vorzugsweise weist der angrenzende Randteil der Öffnung, in welchem zumindest eines der in Längsrichtung gegenüberliegenden Enden der ersten und zweiten elastischen Teile angeordnet ist, eine Führungskerbe zum Aufreißen auf.

Vorzugsweise besteht die zweite obere Schicht aus Vliesstoff und die meisten der Faserbestandteile davon sind in der Richtung ausgerichtet, in der die Führungskerbe aufgerissen wird.

### Kurze Beschreibung der Zeichnung

Die Erfindung wird anhand eines Beispiels in Verbindung mit den zugehörigen Zeichnungen beschrieben, wobei die Figuren folgende Darstellungen zeigen:
- Fig. 1: eine perspektivische Ansicht, der Innenseite einer nach einer Ausführung der Erfindung aufgebauten Wegwerfwindel;
- Fig.2: eine perspektivische Ansicht dieser Ausführungsform geschnitten entlang einer Linie 2-2 in Fig. 1;
- Fig. 3: eine diese Ausführungsform in vergrößertem Maßstab zeigende geschnittene Ansicht entlang der Linie 2-2 in Fig. 1;
- Fig. 4: eine Draufsicht, welche in der endlosen zweiten oberen Schicht angeordnete Öffnungen, wie auch den ersten Schritt des ersten Verfahrens zum Anheften der elastischen Teile an den äußeren Rändern der entsprechenden Öffnungen zeigt;
- Fig. 5: eine Draufsicht, welche in der endlosen zweiten oberen Schicht angeordnete Öffnungen, wie auch den zweiten Schritt des ersten Verfahrens zum Anheften der elastischen Teile am äußeren Rand der entsprechenden Öffnungen zeigt;
- Fig. 6: eine Draufsicht, welche in der endlosen zweiten oberen Schicht angeordneten Öffnungen, wie auch den ersten Schritt des zweiten Verfahrens zum Anheften der elastischen Teile an den äußeren Rändern der entsprechenden Öffnungen zeigt;
- Fig. 7: eine Draufsicht, welche in der endlosen zweiten oberen Schicht angeordnete Öffnungen, wie auch den zweiten Schritt des zweiten Verfahrens zum Anheften der elastischen Teile an die äußeren Ränder der entsprechenden Öffnungen zeigt; und
- Fig. 8: eine Draufsicht, welche in einem vergrößerten Maßstab eine der in Längsrichtung gegenüberliegenden Enden der Öffnung und den an dieses Ende angrenzenden Bereich zeigt.

### Bevorzugte Ausführungsformen der Erfindung

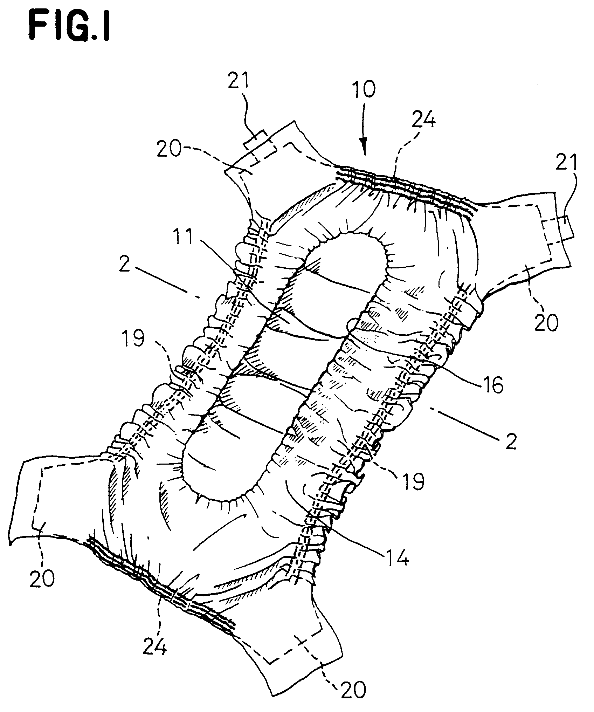

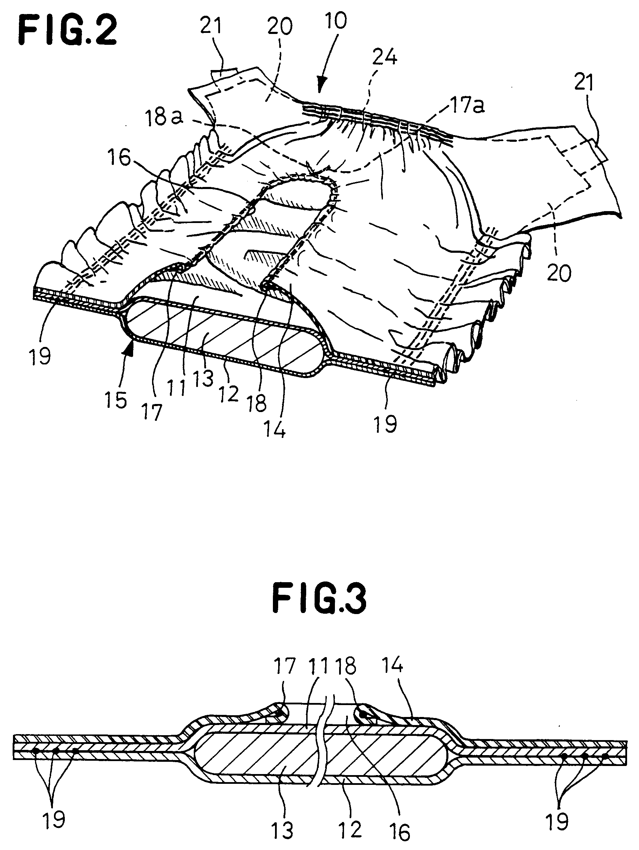

Bezugnehmend auf die Fig. 1 und 2 umfaßt eine Windel 10 ein Integral-Laminat 15 bestehend aus einer flüssigkeitsdurchlässigen ersten oberen Schicht 11, einer flüssigkeitsundurchlässigen unteren Schicht 12, einem flüssigkeitsabsorbierenden Kern 13, der dazwischen eingelegt ist, und einer flüssigkeitsabweisenden zweite "obere" Schicht 14. Die zweite obere Schicht 14 ist in ihrem Zentralbereich mit einer Öffnung 16 ausgebildet, welche in der Längsrichtung länger als in der Querrichtung dieser Schicht 14 ist und in Längsrichtung gegenüberliegende, kreisbogenbeschreibende Enden hat. Die Öffnung 16 kann zumindest innerhalb des Schrittbereiches ausbildet sein.

Unter der Annahme, daß die Öffnung 16 durch eine imaginäre, in Längsrichtung verlaufende zentrale Linie in zwei, eine linke und eine rechte Hälfte geteilt ist, welche entlang und angrenzend an ihren Rändern mit in Längsrichtung dehnbaren ersten und zweiten elastischen Teilen 17, 18 unter Verwendung eines nicht dargestellten Heißschmelzklebemittels angeordnet sind, wobei jedes der ersten und zweiten elastischen Teile 17, 18 eine Vielzahl von elastischen Fäden aufweist, überlappen in Längsrichtung gegenüberliegende Enden 17a der ersten elastischen Teile 17 die zugehörigen Enden des zweiten elastischen Teils 18 in der Nähe der in Längsrichtung gegenüberliegenden Enden der Öffnung 16 (Vgl. Fig.en 2 und 4). Auf diese Weise kann nicht nur ein gesamter äußerer Rand der Öffnung 16 gemeinsam unter der Wirkung dieser elastischen Teile 17, 18 kontrahiert werden, um Aufnahmen zu bilden, sondern können auch die überlappenden Enden dieser elastischen Teile 17, 18 in einfacher Weise voneinander getrennt werden, wenn die zweite obere Schicht 14 vom angrenzenden Bereich dieser überlappenden Enden der elastischen Teile 17, 18 abgezogen wird, da die letzteren nicht endlose schleifenartige, sondern nur sich an ihren in Längsrichtung gegenüberliegenden Enden überlappende elastische Teile sind. Genauer gesagt sind die in Längsrichtung gegenüberliegenden Enden des ersten elastischen Teils 17 an den zugehörigen Enden des zweiten elastischen Teils 18 mittels des Klebemittels gebunden, so daß die beiden zusammengehörenden Enden sich jeweils überlappen, oder sich in seitlichem Kontakt zueinander erstrecken. Dennoch weist der normalerweise zum Verbinden verschiedener Bestandteile der Windel diesen Typs verwendete heißschmelzende Kleber keine ausreichende Haltekraft auf, um das Entfernen zu verhindern, nachdem er auf diese Teile aufgetragen und ausgehärtet ist.

Obwohl nicht dargestellt, können die sich in Längsrichtung gegenüberliegenden Enden des ersten elastischen Teils 17 parallel zu den zugehörigen Enden des zweiten elastischen Teils 18 in Kontakt miteinander oder mit einem schmalen Raum dazwischen, beispielsweise von weniger als 10 mm, erstrecken, soweit der gesamte äußere Rand der Öffnung elastisch kontrahiert werden kann, um im wesentlichen gleichförmige Aufnahmen zu bilden. Es ist auch möglich, ohne den Schutzbereich der Erfindung zu verlassen, und obwohl nicht im einzelnen dargestellt, die zusammengehörigen Enden der ersten und zweiten elastischen Teile, welche einander überlappen können, parallel erstreckend in Kontakt miteinander oder mit einem kleinen Abstand voneinander an den Mittelpunkten der gegenüberliegenden Seitenränder der Öffnung 16 anzuordnen.

Zwischen den gegenüberliegenden Seitenrändern der ersten oberen Schicht 11 und den gegenüberliegenden Seitenrändern der unteren Schicht 12, welche sich beide vom flüssigkeitsabsorbierenden Kern 13 nach außen erstrecken, sind eine Vielzahl von elastischen Teilen 19 befestigt, welche wiederum eine Vielzahl von elastischen Gummifäden aufweisen, die in ihrer gedehnten Form mittels nicht dargestellter Heißschmelzklebemittel befestigt sind, um in Längsrichtung der Schichten dehnbar zu sein und leicht um die Beine des Benutzers zu passen. In ähnlicher Weise sind zwischen den in Längsrichtung gegenüberliegenden Enden der ersten oberen Schicht 11 und den zugehörigen Enden der unteren Schicht 12 eine Vielzahl von elastischen Teilen 24 angeordnet, wobei jedes wiederum eine Vielzahl von elastischen Gummifäden aufweist, um in Querrichtung der Schichten dehnbar zu sein und leicht um die Taille des Benutzers zu passen.

Die erste obere Schicht 11 kann aus Vliesstoff, poröser Kunststoffolie oder ähnlichem bestehen, die untere Schicht 12 kann aus Kunststoffolie, einer laminierten Schicht dieser Kunststoffolie und Vliesstoff oder ähnlichem bestehen, der flüssigkeitsabsorbierende Kern 13 kann aus einer Mischung aus Zellstofflocken und hochabsorbierenden Polimerpuder oder ähnlichem bestehen. Die zweite obere Schicht 14 besteht vorzugsweise aus wasserabstoßendem und hochgradig luftdurchlässigem Vliesstoff. Es ist selbstverständlich, daß in der Beschreibung der Erfindung das "flüssigkeitsabweisende" Material sich auf das Material bezieht, welches ein ausreichendes Maß von Wasserreflexion hat, um zu verhindern, daß flüssige Ausscheidungen in einfacher Weise durchtreten können, wenn die Windel an dem Körper des Benutzers angeordnet ist.

Bezugnehmend auf Fig. 1 hat die Windel 10 zwei Paar flügelähnliche Klappen 20, welche sich von den gegenüberliegenden Seiten der Taillenlinie nach außen erstrecken, und die freien Enden von an den zugehörigen hinteren Seitenflügelklappen 20 befestigten Klebebandbefestiger 21 können klebend an der unteren Schicht 12 befestigt werden, um die Windel um den Körper des Benutzers anzuordnen.

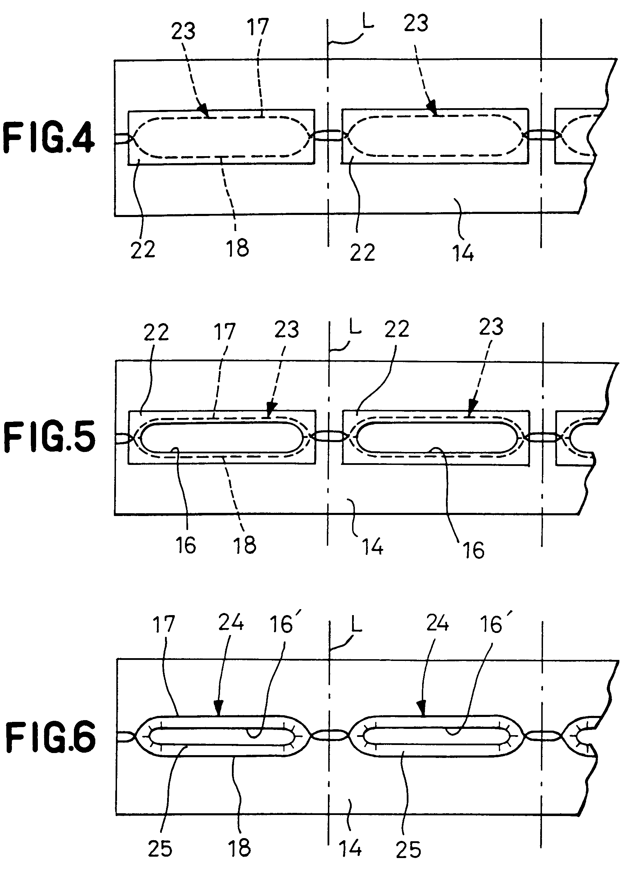

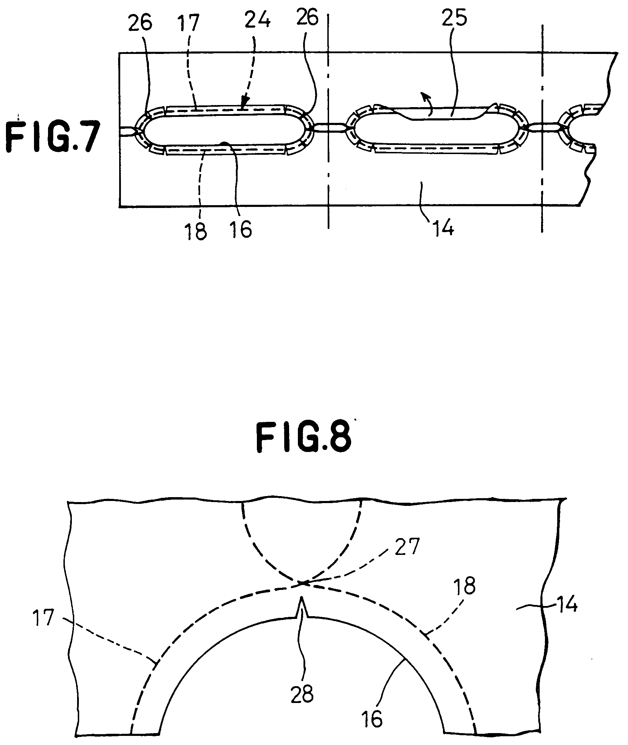

Im folgenden werden bezugnehmend auf die Fig. 4 und 5, die exemplarisch ein erstes Verfahren und die Fig. 6 und 7, welche exemplarisch ein zweites Verfahren zeigen zwei Verfahren zur Ausbildung der Öffnungen 16 in der zweiten oberen Schicht 14 und zum Ausbilden dieser Öffnungen 16 mit den ersten und zweiten elastischen Teilen 17, 18 beschrieben .

Bezugnehmend auf Fig. 4 werden die endlosen ersten und zweiten elastischen Teile 17, 18, welche durch einen vorbestimmten Streckungsprozentsatz gestreckt sind, auf der Rückseite der zweiten oberen Schicht 14 entlang ihrer zentralen Zone mittels eines Paares von nicht dargestellten Querbefestigungsmitteln plaziert und gleichzeitig mittels Heißschmelzklebemitteln daran gebunden, so daß diese zwei elastischen Teile sich periodisch in angrenzenden Bereichen der in Längsrichtung gegenüberliegenden Enden der einzelnen Windeln überlappen. Ein Streifen 22 eines flüssigkeitsresistenten Vliesstoffes von ausreichender Größe um die Länge der ersten und zweiten elastischen Teile 17, 18 abzudecken, welche auf der zweiten oberen Schicht 14 einer jeden einzelnen Windel angeordnet sind, wird auf die zweite obere Schicht 14 mittels Heißschmelzklebemitteln aufgeklebt. Danach werden, wie es durch Fig. 5 erkennbar wird, die zweite obere Schicht 14 und der Streifen 22 aus Vliesstoff teilweise entlang der Linie weggeschnitten, welche sich leicht innerhalb einer elastischen ringförmigen, durch die ersten und zweiten elastischen Teile 17, 18 definierten Linie 23 erstreckt, um die Öffnung 16 mit einer Form zu bilden, welche ähnlich der Form ist, die durch die elastische ringförmige Linie 23 gebildet ist.

Es wird nun auf Fig. 6 Bezug genommen: Endlose erste und zweite elastische Teile 17, 18, welche mit einem vorbestimmten Streckungsprozentsatz gestreckt sind, werden auf der Rückseite der zweiten oberen Schicht 14 entlang ihres Zentralbereichs mittels eines Paares nicht dargestellter Querbefestigungsmitteln plaziert und gleichzeitig darauf mittels Heißschmelzklebemitteln angeklebt, so daß diese zwei elastischen Teile sich periodisch in angrenzenden Bereichen der in Längsrichtung gegenüberliegenden Enden einzelner Windeln überlappen. Dann wird die zweite obere Schicht 14 teilweise entlang der Linie weggeschnitten, welche sich leicht innerhalb einer elastischen ringförmigen Linie 24 erstreckt, die durch die ersten und zweiten elastischen Teile 17, 18 definiert ist, um eine Öffnung 16 mit einer Form zu bilden, welche ähnlich der Form ist, die durch die elastische ringförmige Linie 24 definiert ist. Anschließend wird, wie es durch die Fig. 7 dargestellt ist, ein äußerer Rand 25, der sich nach innen gerichtet von der elastischen ringförmigen Linie 24 erstreckt, zurückgefaltet und an der zweiten oberen Schicht 14 mittels Heißschmelzklebermitteln zur Überdeckung der elastischen ringförmigen Linie 24 angeheftet, um dabei die gewünschte Öffnung 16 zu bilden. Um sicherzustellen, daß die in Längsrichtung gegenüberliegenden runden bogenförmigen Enden der Öffnung 16 während des Zurückfallens des Umfangsrandes sauber zurückgefaltet werden können, weisen diese kreisbogenförmigen Enden Kerben 26 in regelmäßigen Abständen auf.

Bezugnehmend auf die Fig. 4 bis 7 bezeichnet der Bezugsbuchstabe L eine imaginäre Linie, entlang welcher die zweite obere Schicht 14 geschnitten wird, um einzelne Windeln zu erhalten. Dieser Schneidevorgang wird durch Schneiden des endlosen Laminates in die einzelnen Windeln bewirkt, nachdem derartiges endloses Laminat aufgebaut wurde (Vgl. das Laminat 15, wie es in Fig. 2 dargestellt ist). Es ist selbstverständlich, daß die Längen der ersten und zweiten sich zwischen jedem Paar der angrenzenden elastischen ringförmigen Linien 23 (24) erstreckenden elastischen Teile 17, 18 mit oder ohne Klebemittel versehen werden können und daß, wenn kein Klebemittel verwendet wird, diese Längen der elastischen Teile 17, 18 zum Zeitpunkt des Schneidens in geeigneter Weise zurückgeschnappt sind.

Der äußere Rand der Öffnung 16 wird in Bereichen, die gegenüberliegend der und angrenzend an dem Schnittpunkt der ersten und zweiten elastischen Teile 17, 18 angeordnet sind, mit Führungskerben 28 zum Aufreißen versehen. Diese Führungskerbe 28 ist vorzugsweise an jedem der in Längsrichtung gegenüberliegenden Enden der Öffnung 16 angeordnet, kann aber auch an jedem beliebigen Ende angeordnet sein. Während diese Führungskerbe 28 sehr gut dazu geeignet ist, das Abreißen der zweiten oberen Schicht 14 zu ermöglichen, kann das Abreißen der zweiten oberen Schicht 14 darüberhinaus vereinfacht werden durch vorzugsweises Orientieren der Faserbestandteile der zweiten oberen Schicht 14 in die Richtung, entlang welcher die Führungskerbe zu ziehen ist, beispielsweise in Längsrichtung der Windel gemäß dem dargestellten Ausführungsbeispiel, wenn die letztere (zweite obere Schicht 14) aus Vliesstoff besteht.

Während die Windel des sogenannten offenen Typs, welche die Klebebandbefestiger zum Verschließen der Taillenlinie verwenden, als eine spezielle Ausführungsform dargestellt und beschrieben wurde, kann die Erfindung auch bei Windeln des sogenannten Hosentyps (einschließlich der Trainigshosen) verwendet werden, welche eine endlose Taillenlinie haben.

Flüssige Ausscheidungen werden durch die Öffnung 16 und dann die erste obere Schicht 11 in den Kern 13 absorbiert, während feste Ausscheidungen in eine Tasche fließen, die zwischen der ersten oberen Schicht 11 und der zweiten oberen Schicht 14 ausgebildet ist. Derartige feste Ausscheidungen können, falls erforderlich, entfernt werden, nachdem die zweite obere Schicht 14 von der Führungskerbe 28 abgerissen wurde, um die erste obere Schicht 11 über eine entsprechende Ausdehnung freizulegen.

Gemäß der Erfindung sind die ersten und zweiten elastischen Teile unabhängig an der zweiten oberen Schicht im wesentlichen entlang der Hälften der Öffnung befestigt, so daß im wesentlichen der gesamte äußere Rand durch diese ersten und zweiten elastischen Teile elastisch wird. Dennoch, wie durch die voranstehende Beschreibung erkennbar wird, sind diese ersten und zweiten elastischen Teile nicht miteinander verbunden, sondern überlappend, berührend oder sich aneinander annähernd an ihren längsseitig gegenüberliegenden Enden angeordnet. Diese Anordnung ermöglicht, daß die in Längsrichtung gegenüberliegenden Enden der ersten und zweiten elastischen Teile in einfacher Weise voneinander getrennt werden, wenn die zweite obere Schicht von den an diese Enden angrenzenden Bereiche entfernt wird, wodurch die erste obere Schicht, welche unterhalb der zweiten oberen Schicht liegt, freigelegt werden kann, so daß feste Ausscheidungen, welche an der ersten oberen Schicht anhaften, entfernt werden können.

Die voranstehend beschriebene Vorgehensweise des Abreißens der zweiten oberen Schicht wird vereinfacht durch Anordnung der Führungskerbe für derartiges Abreißen in die Randabschnitte der Öffnung, neben den Bereichen, an denen die in Längsrichtung gegenüberliegenden Enden der ersten und zweiten elastischen Teile sich schneiden (überkreuzen), sich berühren oder sich aneinander annähern, und ferner erleichtert durch Ausrichten der meisten Faserbestandteile des als Material für die zweite Oberschicht verwendeten Vliesstoffes in die Richtung, entlang welcher die Führungskerbe aufgerissen wird.

## Patentansprüche

1. Wegwerfwindel mit einem Integral-Laminat (15), welches zusammengesetzt ist aus einer flüssigkeitsdurchlässigen ersten oberen Schicht (11), einer flüssigkeitsundurchlässigen unteren Schicht (12), einem flüssigkeitsabsorbierenden Kern (13), der zwischen der ersten oberen Schicht (11) und der unteren Schicht (12) eingelegt ist, und einer flüssigkeitsabweisenden, über der ersten oberen Schicht (11) liegenden zweiten oberen Schicht (14), wobei die zweite obere Schicht (14) in ihrem zentralen Bereich im wesentlichen mit einer Öffnung (16) versehen ist, welche sich in der Längsrichtung länger erstreckt als in der Querrichtung der zweiten oberen Schicht (14); wobei die zweite obere Schicht (14) entlang ihres äußeren Begrenzungsrandes mit der ersten obere Schicht (11) verbunden ist; und wobei in Längsrichtung elastische Teile (17,18) an der zweiten oberen Schicht (14) entlang des äußeren Randes der Öffnung (16) angeordnet sind,
**dadurch gekennzeichnet,**
daß die elastischen Teile erste und zweite elastischen Teile (18,17) aufweisen, welche nicht miteinander verbunden sind, und
daß im wesentlichen der gesamte Rand der Öffnung (16) elastisch gemacht ist durch das Anbringen dieser ersten und zweiten elastischen Teile (18,17) im wesentlichen entlang der jeweiligen Hälften des Randes.

2. Wegwerfwindel nach Anspruch 1, bei der sich in Längsrichtung gegenüberliegende Enden der ersten und zweiten elastischen Teile (17,18) an den in Längsrichtung gegenüberliegenden Enden der Öffnung (16) überlappen.

3. Wegwerfwindel nach Anspruch 1, bei der sich die in Längsrichtung gegenüberliegenden Enden der ersten und zweiten elastischen Teile (17,18) an den in Längsrichtung gegenüberliegenden Enden der Öffnung (16) berühren.

4. Wegwerfwindel nach Anspruch 1, bei der sich die in Längsrichtung gegenüberliegenden Enden der ersten und zweiten elastischen Teile (17,18) an den in Längsrichtung gegenüberliegenden Enden der Öffnung (16) annähern.

5. Wegwerfwindel nach Anspruch 1, bei der die ersten und zweiten elastischen Teile (17,18) mit dem äußeren Rand der Öffnung (16) bedeckt sind.

6. Wegwerfwindel nach Anspruch 1, bei der jedes der ersten und zweiten elastischen Teile (17,18) mindestens einen Gummifaden aufweist.

7. Wegwerfwindel nach Anspruch 1, bei der der angrenzende Randteil der Öffnung (16) in welchem zumindest eines der in Längsrichtung gegenüberliegenden Enden der ersten und zweiten elastischen Teile (17,18) angeordnet ist, eine Führungskerbe (28) zum Aufreißen aufweist.

8. Wegwerfwindel nach Anspruch 7, bei der die zweite obere Schicht (14) aus Vliesstoff besteht und die meisten der Faserbestandteile davon in Richtung, in der die aufzureißende Führungskerbe (28) aufzureißen ist, ausgerichtet sind.

## Claims

1. A disposable nappy with an integral laminate (15) which is composed of a first upper sheet (11) which is permeable to liquids, a lower sheet (12) which is impermeable to liquids, a liquid-absorbing core (13) which is disposed between the first upper sheet (11) and the lower sheet (12), and a liquid-repellant second upper sheet (14) lying over the first upper sheet (11), wherein the second upper sheet (14) is provided with an opening (16) substantially in its central region which extends further in the longitudinal direction than in the transverse direction of the second upper sheet (14); wherein the second upper sheet (14) is connected to the first upper sheet (11) along its outer bounding edge; and wherein elastic parts (17, 18) are arranged on the second upper sheet (14) in the longitudinal direction along the outer edge of the opening (16), characterized in that the elastic parts comprise first and second elastic parts (17, 18) which are not joined to one another and in that substantially the whole of the edge of the opening (16) is elasticated by the application of these first and second elastic parts (17, 18) substantially along the respective halves of the edge.

2. A disposable nappy according to claim 1, in which opposite ends in the longitudinal direction of the first and second elastic parts (17, 18) overlap at the opposite ends on the longitudinal direction of the opening (16).

3. A disposable nappy according to claim 1, in which the opposite ends in the longitudinal direction of the first and second elastic parts (17, 18) touch each other at the opposite ends in the longitudinal direction of the opening (16).

4. A disposable nappy according to claim 1, in which the opposite ends in the longitudinal direction of the first and second elastic parts (17, 18) approach each other at the opposite ends in the longitudinal direction of the opening (16).

5. A disposable nappy according to claim 1, in which the first and second elastic parts (17, 18) are covered by the outer edge of the opening (16).

6. A disposable nappy according to claim 1, in which each of the first and second elastic parts (17,18) comprises at least one rubber thread.

7. A disposable nappy according to claim 1, in which the adjoining edge part of the opening (16) in which is arranged at least one of the ends of the first and second elastic parts (17, 18) opposite one another in the longitudinal direction has a lead-in nick (28) for tearing apart.

8. A disposable nappy according to claim 7, in which the second upper sheet (14) consists of fleece material and the majority of the fibre components thereof are aligned in a direction in which the lead-in nick for tearing apart (28) is to be torn apart.

## Revendications

1. Lange jetable comportant un lamifié intégral (15) composé d'une première couche supérieure perméable aux liquides (11), d'une couche inférieure imperméable aux liquides (12), d'un corps central absorbant les liquides (13), emprisonné entre la première couche supérieure (11) et la couche inférieure (12), et d'une seconde couche supérieure hydrofuge (14) disposées au-dessus de la première couche supérieure (11), ladite seconde couche supérieure (14) étant pourvue, en substance, dans sa zone centrale, d'une ouverture (16) qui présente une dimension plus importante dans le sens de la longueur que dans le sens transversal de la seconde couche supérieure (14); la seconde couche supérieure (14) étant jointe a la première couche supérieure (11) le long de sa lisière extérieure; lange dans lequel des éléments élastiques (17,18) sont appliqués longitudinalement sur la seconde couche supérieure (14), le long du bord extérieur de l'ouverture (16), et caractérisé en ce que les éléments élastiques comprennent des premiers et des seconds éléments élastiques (18,17), qui ne sont pas liés les uns aux autres, et en ce que pour ainsi dire tout le bord de l'ouverture (16) est rendu élastique en posant ces premiers et seconds éléments élastiques (18,17) pour l'essentiel le long des moitiés appropriées du bord.

2. Lange jetable selon la revendication 1, dans lequel des extrémités des premiers et seconds éléments élastiques (17,18), opposées dans le sens longitudinal, se chevauchent aux extrémités de l'ouverture (16) opposées dans le sens de la longueur.

3. Lange jetable selon la revendication 1, dans lequel des extrémités des premiers et seconds éléments élastiques (17,18), opposées dans le sens longitudinal, se touchent aux extrémités de l'ouverture (16) opposées dans le sens de la longueur.

4. Lange jetable selon la revendication 1, dans lequel des extrémités des premiers et seconds éléments élastiques (17,18), opposées dans le sens longitudinal, se rapprochent aux extrémités de l'ouverture (16) opposées dans le sens de la longueur.

5. Lange jetable selon la revendication 1, dans lequel les premiers et seconds éléments élastiques (17,18) sont recouverts par le bord extérieur de l'ouverture (16)

6. Lange jetable selon la revendication 1, dans lequel chacun des premiers et seconds éléments élastiques (17,18) présente au moins un fil de caoutchouc.

7. Lange jetable selon la revendication 1, dans lequel la zone limitrophe de l'ouverture (16), dans laquelle se trouve au moins l'une des extrémités des premier et second éléments élastiques (17,18) opposées dans le sens longitudinal, présente une encoche de guidage (28) facilitant le déchirement.

8. Lange jetable selon la revendication 7, dans lequel la seconde couche supérieure (14) est fabriquée dans un non tissé et ou la majeure partie des fibres composant cette dernière sont orientées dans la direction dans laquelle il convient d'ouvrir l'encoche de guidage (28) devant être ouverte par déchirement.
